Europäisches Patentamt

(19) European Patent Office

Office européen des brevets

(11) Publication number: **0 001 905**
**B1**

(12) **EUROPEAN PATENT SPECIFICATION**

(45) Date of publication of patent specification: **02.09.81**

(51) Int. Cl.³: **C 07 C 21/21, C 07 C 17/34**

(21) Application number: **78300551.5**

(22) Date of filing: **27.10.78**

(54) **Production of chloroprene.**

(30) Priority: **29.10.77 GB 4513977**

(43) Date of publication of application:
**16.05.79 Bulletin 79/10**

(45) Publication of the grant of the European patent:
**02.09.81 Bulletin 81/35**

(84) Designated Contracting States:
**DE FR GB**

(56) References cited:
**FR - A - 2 259 076**
**FR - A - 2 341 545**
**GB - A - 1 173 359**

**CHEMICAL ABSTRACTS, vol. 87, 22279w, 1977,
page 568. Columbus, Ohio, USA.
SHOSTAKOVSKII et al.: "Use of two-phase
catalytic systems in the dehydrohalogenation of
β-haloethyl ethers".**

(73) Proprietor: **BP Chemicals Limited**
**Britannic House Moor Lane**
**London, EC2Y 9BU (GB)**

(72) Inventor: **Hall, Anthony Harold Patrick**
**BP Research Centre Chertsey Road**
**Sunbury-on-Thames Middlesex, TW16 7LN (GB)**

(74) Representative: **Ryan, Edward Terrence et al,**
**BP INTERNATIONAL LIMITED Patents and
Licensing Division Chertsey Road
Sunbury-on-Thames Middlesex, TW16 7LN (GB)**

Courier Press, Leamington Spa, England.

## Production of chloroprene

The present invention relates to a process for the dehydrochlorination of 3,4-dichlorobutene-1 to give chloroprene (2-chlorobutadiene).

It is disclosed in French patent specification FR-A-2341545 that chloroprene may be produced by continuously dehydrochlorinating 3,4-dichlorobutene-1 at a temperature in the range 40°—70°C in a reaction medium which is a two phase liquid mixture of water and an alcohol in a volume ratio of water to alcohol from 90:10 to 5:95 in the presence of a standing concentration of not more than 10% by weight of alkali metal hydroxide in the aqueous phase, and recovering a liquid phase containing chloroprene from the reaction mixture.

The preferred alcohol in the French patent specification is 2-butoxyethanol, which gives a fast rate of reaction. However there is a slight loss of alcohol in the process, and 2-butoxyethanol is more expensive than the simple $C_3$ or $C_4$ alcohols, such as n-butanol andiso-butanol. Reaction rates are much slower when using these cheaper alcohols. In order to obtain chloroprene production rates suitable for commercial operation it is necessary to use either a large excess of alkali metal hydroxide or a large number of reactors in series.

Chemical Abstracts vol 87, 22279W, 1977 page 568 discloses the use of a two phase catalytic system for the dehydrohalogenation of $\beta$-haloethyl ethers at 140—50°C using highly concentrated 50—85% aqueous KOH, a quaternary ammonium compound and dimethyl sulphone. Such a system using an expensive solvent such as dimethyl sulphone and KOH solutions of extremely high concentration would not be suitable for large scale industrial production of a commercial monomer.

GB 1 173 359 describes and claims a process for producing an unsaturated aliphatic compound (e.g. chloroprene) which comprises dehydrohalogenating a halogenated aliphatic compound containing at least two carbon atoms (e.g. 3,4-dichlorobutene-1) with an inorganic alkaline material in an aqueous medium in the presence of a quaternary ammonium compound. The dehydrochlorination may be carried out at temperatures in the range 40° to 85°C e.g. 60°C. There is however no disclosure of carrying out the dehydrochlorination in the presence of an alcohol.

A person skilled in the art would believe that the process of GB 1 173 359 takes place in the aqueous phase in view of the known variation in reaction rate with NaOH concentration in reactions of this type. The process of FR 2 341 545 takes place in the organic phase (see US 3 965 203). As the process of GB 1 173 359 takes place in the aqueous phase the expensive quaternary ammonium compound which is known to be water-soluble would be expected to be found in the aqueous phase. The man skilled in the art would expect that there would be a considerable proportion of the quaternary ammonium compound present in the aqueous phase. He would therefore expect that if a continuous process based on a quaternary ammonium catalyst is to be economically feasible it will be necessary either to recycle the aqueous phase, which would involve considerable difficulty and expense in removing the sodium chloride reaction product, or to find some method of recovering the quaternary ammonium compound from the aqueous phase, which is also likely to be difficult and expensive. The removal of the quaternary ammonium is also desirable if the aqueous phase is to be fed to a biological effluent treatment step, as many quaternary ammonium compounds have a biocidal action.

The person skilled in the art would see good reasons to avoid the use of the process of GB 1 173 359. He would see no good reason to combine the disclosure of GB 1 173 359 on processes carried out in the aqueous phase with the disclosure of FR 2 341 545 relating to dehydrochlorination in the organic phase. He would see no good reason to believe that such a combination would overcome the problems of loss of quaternary ammonium compound in the aqueous effluent.

It has now, surprisingly, been found that a continuous dehydrochlorination process can be carried out without the necessity of recycling the aqueous phase or recovering quaternary ammonium compound from the aqueous phase.

According to the invention a process for the production of chloroprene by the continuous dehydrochlorination of 3,4-dichlorobutene in the presence of aqueous alkali metal hydroxide in a reaction mixture which is a two phase liquid mixture of water and alcohol having 3 or 4 carbon atoms in the molecule in a volume ratio of water to alcohol from 90:10 to 5:95 in the presence of a standing concentration of not more than 10% by weight of alkali metal hydroxide in the aqueous phase followed by separation of an organic liquid phase containing chloroprene from the resulting mixture of aqueous and organic phases, separation of chloroprene from the organic phase which is recycled to the dehydrochlorination is characterised in that the dehydrochlorination reaction is carried out at a temperature in the range 40°—80°C in the presence of a quaternary ammonium compound and the aqueous phase is discarded without recovering quaternary ammonium compound from the aqueous phase.

The process of the present invention is carried out continuously. The reaction may be carried out in a stirred reaction vessel to which 3,4-dichlorobutene-1, water, alcohol and alkali metal hydroxide are fed continuously and from which liquid reaction mixture containing chloroprene is continuously withdrawn. A plurality of such stirred reactor vessels in series may be used, with the product from the first reactor in series overflowing into the next reactor.

The reaction temperature is preferably in the range 50°—70°C, for example 55°—65°C. Depending on the amount of reaction taking place in each reactor the temperature may rise adiabatically or may be controlled by cooling. The reaction is most conveniently carried out at atmospheric pressure and when using atmospheric pressure, temperatures much above 70°C tend to cause a substantial amount of chloroprene to boil off from the reactor mixture.

The process of the present invention is most conveniently carried out at pressures close to atmospheric. If the pressure is reduced much below atmospheric pressure the chloroprene may boil at the reaction temperature while the use of pressure much above atmospheric pressure involves additional expense in providing pressure resistant vessels and pumps for maintaining high pressures. In commercial plant it may be necessary to use a pressure somewhat above atmospheric pressure to overcome the back pressure of the distillation system used to recover the chloroprene. It is therefore preferred to carry out the processes at a pressure from 1 atmosphere to $1\frac{1}{2}$ atmospheres absolute, for example at a pressure in the range 1 to $1\frac{1}{4}$ atmospheres absolute.

Examples of suitable alcohols are n-butanol (butanol-1) and iso-butanol (butanol-2).

It is preferred to use less than 50 volumes of water for every 50 volumes of alcohol, more preferably to use a water:alcohol volume ratio of from 40:60 to 10:90.

The process of the present invention is a continuous process and therefore requires both alkali metal hydroxide and 3,4-dichlorobutene-1 to be fed continuously into the reaction medium. The alkali metal hydroxide, preferably sodium hydroxide, is most conveniently fed as an aqueous solution. This aqueous solution may have a concentration of 25% wt/wt sodium hydroxide, or more, but the process is so operated that the standing concentration in the aqueous phase in the reaction medium does not exceed 10% by weight based on weight of aqueous phase. This minimises the risk of precipitation of alkali metal chloride from the reaction medium.

The concentration of the alkali metal hydroxide in the aqueous effluent from the dehydrochlorination reactor preferably does not exceed 4% by weight, more preferably 3% by weight, based on weight of aqueous phase. A low concentration of alkali metal hydroxide in the aqueous phase enables the aqueous phase to be discarded without the necessity of providing a means for recovering and recycling the alkali metal hydroxide. In order to obtain such a low concentration of alkali metal hydroxide in the aqueous effluent from the reaction, it will usually be desirable to employ at least two reactors in series so that the necessary low concentration of alkali metal hydroxide can be maintained in the final reactor.

The 3,4-dichlorobutene-1 and the alkali metal hydroxide are fed to the reaction at a molar ratio of alkali metal oxide to 3,4-dichlorobutene-1 which is preferably not more than 1.3:1, more preferably not more than 1.2:1. In order for the reaction to proceed satisfactorily it will however usually be necessary to have a stoichiometric excess of alkali metal hydroxide.

The rate at which the 3,4-dichlorobutene-1 is fed to the dehydrochlorination reaction is preferably controlled to give a residence time in the reaction system in the range 10 to 60 minutes.

The rate at which 3,4-dichlorobutene-1 is fed to the reactor ion is preferably such as to maintain a 3,4-dichlorobutene-1 concentration in the reactor to which it fed in the range 2 to 15% wt/wt.

The dehydrochlorination is preferably carried out in the presence of a polymerisation inhibitor for example nitric oxide and the sodium hydroxide may contain sodium sulphide.

The process of the present invention is carried out in the presence of a quaternary ammonium compound.

The amount of quaternary ammonium catalyst will depend on the particular compound used, e.g. as little as 0.01% by weight being effective with the most active compounds, and there generally being no economic reason to use more than 10% by weight based on the weight of dehydrochlorination reaction medium.

A considerable range of quaternary ammonium compounds may be used. Examples of preferred compounds, which are readily available commercially are:

tetra-n-butylammonium bromide or hydroxide
cetyltrimethylammonium bromide or chloride (as Arquad 16—50®)
dodecyltrimethylammonium chloride or mix-tures containing this (e.g. Arquad C—50®)
tricaprylylmethylammonium chloride (e.g. Aliquat 336®)

The aqueous phase and organic phase can be separated by decantation. The aqueous phase containing alkali metal chloride and some residual alkali metal hydroxide is discarded and not recycled. It may be possible to use the discarded aqueous phase in another process e.g. electrolysis, or it may be discharged as an effluent.

Chloroprene is most conveniently recovered from the organic phase by distilling it off from the organic phase as a vapour. The residual organic phase containing the alcohol, any unreacted dichlorobutenes and the quaternary ammonium compound is recycled to the dehydrochlorination. It is the surprising fact that the quaternary ammonium compound remains in the organic phase and is not lost in the aqueous phase which makes possible the relatively simple process of the present invention which avoids the need for a complex system for recycling water or recovering quaternary ammonium compound.

## Example 1

3,4-dichlorobutene-1 (225 g/h) and 20% sodium hydroxide solution (432 g/h) were fed continuously to a stirred reactor of 750 ml volume maintained at 64°C. The contents overflowed into a second reactor of 500 ml volume maintained at 70°C. Before commencing the run, the reactors were charged with a mixture of brine and n-butanol to which 17.4 g of cetyltrimethylammonium bromide had been added. The product from the second reactor passed to a decanter from which brine was removed and the organic phase fed to a distillation column. Chloroprene was removed from the head of the column and the product from the base was recycled to the first reactor.

Over a 3.5 hour period 785 g of 3,4-dichlorobutene-1 (99.6% purity) were fed and 542 g (98.8% purity) of chloroprene obtained (97% yield). Under equilibrium conditions the reactors contained approximately 55% by volume organic phase in which the 3,4-dichlorobutene-1 concentrations were 8.3% and 4.8% in the first and second reactors respectively. Similar results were obtained over several runs without the further addition of catalysts, and no quaternary salt could be detected in the effluent brine, the limit of detection being about 2 ppm.

*Comparative Example*

With conditions as described in Example 1 but with no cetyltrimethylammonium bromide present, 214 g of chloroprene were obtained over a period in which 364 g of 3,4-dichlorobutene-1 were fed; equivalent to a yield of only 83%. After only 5.4 hours on stream the reactors contained approximately 75% organic phase in which the 3,4-dichlorobutene-1 concentrations were 24.2% and 20.4% in the first and second reactors respectively, and no equilibrium was achieved.

## Example 2

With reaction temperatures and feed rates as described in Example 1, but with isobutanol in place of n-butanol and with 8.7 g of tetra-butylammonium hydroxide added to the reaction mixture in place of cetyltrimethylammonium bromide, 650 g of chloroprene were obtained over a run period in which 922 g of 3,4-dichlorobutene-1 were fed (98% yield). The reactors contained approximately 65% by volume organic phase which contained 5.0% and 3.6% 3,4-dichlorobutene-1 in the first and second reactors respectively. Similar results were obtained over 3 periods of approximately 6 hours each, and no catalyst was found in the brine effluent.

Under similar conditions without the addition of the quaternary salt a 73% yield of chloroprene was obtained, and the 3,4-dichlorobutene-1 concentration in the organic phase in the second reactor reached 30% after only 4.8 hours on stream.

## Example 3

With iso-butanol containing Arquad C50® (1.8% as dodecyltrimethyl ammonium chloride) in place of n-butanol and cetyltrimethyl bromide and with reaction conditions as described in Example 1, 1085 g of chloroprene were obtained from 1576 g of 3,4-dichlorobutene-1 over a run period of 7 hours. No decrease in concentration of the catalyst in the organic phase was observed over a 15 hour period, and no quaternary salt was detected in the effluent brine. The yield of 1-chlorobutadiene was only 0.8% and the formation of other by-products was small.

## Example 4

With reaction temperatures and conditions as used in Example 1, but with iso-propanol in place of n-butanol and tetra-n-butylammonium hydroxide (1%) in place of cetyltrimethylammonium bromide a 96.4% yield of chloroprene was obtained. In the absence of catalyst, however, conversion of the dichlorobutene was incomplete and the yield fell to only 83%.

In all the above Examples the aqueous solution which was the aqueous phase resulting from the dehydrochlorination step was discharged as brine effluent to the drains.

## Claims

1. A process for the production of chloroprene by the continuous dehydrochlorination of 3,4-dichlorobutene in the presence of aqueous alkali metal hydroxide in a reaction mixture which is a two phase liquid mixture of water and alcohol having 3 or 4 carbon atoms in the molecule in a volume ratio of water to alcohol from 90:10 to 5:95 in the presence of a standing concentration of not more than 10% by weight of metal hydroxide in the aqueous phase followed by separation of an organic liquid phase containing chloroprene from the resulting mixture of aqueous and organic phases, separation of chloroprene from the organic phase, which is recycled to the dehydrochlorination characterised in that the dehydrochlorination reaction is carried out at a temperature in the range 40°—80°C in the presence of a quaternary ammonium compound and the aqueous phase is discarded without recovering quaternary ammonium compound from the aqueous phase.

2. A process according to Claim 1 wherein

## 0 001 905

the dehydrochlorination is carried out at a temperature in the range 50°—70°C.

3. A process according to either of the preceding claims wherein the alcohol is n-butanol or isobutanol.

4. The process according to any one of the preceding claims wherein the dehydrochlorination is carried out with less than 50 volumes of water for every 50 volumes of alcohol.

5. The process according to any one of the preceding claims wherein the water:alcohol volume ratio of from 40:60 to 10:90.

6. The process according to any one of the preceding claims wherein the concentration of alkali metal hydroxide in the aqueous phase removed from the dehydrochlorination is not more than 4% by weight based on the aqueous phase.

7. A process according to any one of the preceding claims wherein the 3,4-dichlorobutene-1 and the alkali metal hydroxide are fed to the dehydrochlorination at a molar ratio of alkali metal hydroxide to 3,4-dichlorobutene-1 which is not more than 1.3:1.

8. A process according to any one of the preceding claims wherein the rate at which the 3,4-dichlorobutene-1 is fed to the reactor is controlled to give a residence time in the reaction system in the range 10 to 60 minutes.

9. A process according to any one of the preceding claims wherein the quantity of quaternary ammonium compound present in the dehydrochlorination reaction sodium is 0.01% to 10% by weight of the dehydrochlorination reaction medium.

## Revendications

1. Procédé de production du chloroprène par la déshydrochloration continue du 3,4-dichlorobutène en présence d'une solution aqueuse d'un hydroxyde de métal alcalin dans un mélange réactionnel, qui est un mélange à deux phases liquides d'eau et d'un alcool comportant 3 ou 4 atomes de carbone dans sa molécule selon un rapport en volume de l'eau à l'alcool compris entre 90:10 et 5:95, en présence d'une concentration constante n'excédant pas 10% en poids de l'hydroxyde de métal alcalin dans la phase aqueuse, suivie de la séparation d'une phase liquide organique, contenant le chloroprène, enlevée du mélange résultant de phases aqueuse et organique, et de la séparation du chloroprène de la phase organique qui est recyclée vers la déshydrochloration, ce procédé étant caractérisé en ce qu'on effectue la réaction de déshydrochloration à une température comprise entre 40° et 80°C en présence d'un composé d'ammonium quaternaire et en ce qu'on écarte la phase aqueuse, sans récupérer ou extraire le composé d'ammonium quaternaire de la phase aqueuse.

2. Procédé selon la revendication 1, caractérisé en ce qu'on effectue la déshydrochloration à une température comprise entre 50° et 70°C.

3. Procédé selon l'une des revendications 1 et 2, caractérisé en ce que l'alcool est le n-butanol ou l'isobutanol.

4. Procédé selon l'une quelconque des revendications précédentes, caractérisé en ce qu'on effectue la déshydrochloration avec une proportion de moins de 50 volumes d'eau pour 50 volumes d'alcool.

5. Procédé selon l'une quelconque des revendications précédentes, caractérisé en ce que le rapport en volume entre l'eau et l'alcool se situe entre 40:60 et 10:90.

6. Procédé selon l'une quelconque des revendications précédentes, caractérisé en ce que la concentration de l'hydroxyde de métal alcalin dans la phase aqueuse retirée de la déshydrochloration n'est pas supérieure à 4% en poids par rapport à la phase aqueuse.

7. Procédé selon l'une quelconque des revendications précédentes, caractérisé en ce que le 3,4-dichlorobutène-1 et l'hydroxyde de métal alcalin sont introduits dans la réaction de déshydrochloration selon un rapport molaire entre l'hydroxyde de métal alcalin et le 3,4-dichlorobutène-1 qui n'est pas supérieur à 1,3:1.

8. Procédé selon l'une quelconque des revendications précédentes, caractérisé en ce que le débit d'introduction du 3,4-dichlorobutène-1 dans le réacteur est réglé de manière à obtenir une durée de séjour dans le système de réaction se situant entre 10 et 60 minutes.

9. Procédé selon l'une quelconque des revendications précédentes, caractérisé en ce que la quantité du composé d'ammonium quaternaire présent dans le milieu de la réaction de déshydrochloration représente 0,01% à 10% du poids de ce milieu de réaction de déshydrochloration.

## Patentansprüche

1. Verfahren zur Herstellung von Chloropren durch kontinuierliche Dehydrochlorierung von 3,4-Dichlorbuten in Anwesenheit von wäss-rigem Alkalimetallhydroxid in einer Reaktions-mischung, die eine flüssige zweiphasige Mischung aus Wasser und einem Alkohol mit 3 oder 4 Kohlenstoffatomen im Molekül in einem Volumenverhältnis von Wasser zu Alkohol von 90:10 bis 5:95 darstellt, in Anwesenheit einer ständigen Konzentration an Alkalimetallhydroxid von nicht mehr als 10 Gew.-% in der wässrigen Phase und anschließende Abtrennung einer organischen, flüssigen, Chloropren enthaltenden Phase aus der erhaltenen Mischung aus wässriger und organischer Phase, Abtrennung von Chloropren aus der organischen Phase, die zur Dehydrochlorierung zurückgeführt wird, dadurch gekennzeichnet, daß die Dehydrochlorierungsreaktion bei einer Temperatur zwischen 40 bis 80°C in Anwesenheit einer quaternären Ammoniumverbindung durchgeführt und die wässrige Phase ohne Rückgewinnung der quaternären Ammoniumverbindung aus der wässrigen Phase verworfen wird.

2. Verfahren nach Anspruch 1, dadurch gekennzeichnet, daß die Dehydrochlorierung bei einer Temperatur zwischen 50 bis 70°C durchgeführt wird.

3. Verfahren nach einem der vorhergehenden Ansprüche, dadurch gekennzeichnet, daß der Alkohol n-Butanol oder Iso-butanol ist.

4. Verfahren nach einem der vorhergehenden Ansprüche, dadurch gekennzeichnet, daß die Dehydrochlorierung mit weniger als 50 Volumen Wasser pro 50 Volumen Alkohol durchgeführt wird.

5. Verfahren nach einem der vorhergehenden Ansprüche, dadurch gekennzeichnet, daß das Volumenverhältnis von Wasser zu Alkohol 40:60 bis 10:90 beträgt.

6. Verfahren nach einem der vorhergehenden Ansprüche, dadurch gekennzeichnet, daß die Konzentration des Alkalimetallhydroxids in der wässrigen, aus der Dehydrochlorierung entfernten Phase nicht mehr als 4 Gew.-%, bezogen auf die wässrige Phase, beträgt.

7. Verfahren nach einem der vorhergehenden Ansprüche, dadurch gekennzeichnet, daß 3,4-Dichlorbuten-1 und Alkalimetallhydroxid in einem molaren Verhältnis von Alkalimetallhydroxid zu 3,4-Dichlorbuten-1 von nicht mehr als 1,3:1 zur Dehydrochlorierung geführt werden.

8. Verfahren nach einem der vorhergehenden Ansprüche, dadurch gekennzeichnet, daß die Geschwindigkeit, mit welcher das 3,4-Dichlorbuten-1 in den Reaktor eingeführt wird, so geregelt wird, daß sich eine Verweilzeit im Reaktionssystem zwischen 10 bis 60 Minuten ergibt.

9. Verfahren nach einem der vorhergehenden Ansprüche, dadurch gekennzeichnet, daß die im Reaktionsmedium für die Dehydrochlorierung anwesende Menge an quaternärer Ammoniumverbindung 0,01 bis 10 Gew.-% des Reaktionsmediums für die Dehydrochlorierung beträgt.